# EUROPEAN PATENT SPECIFICATION

(11) **EP 2 859 912 B1**
(45) Date of publication and mention of the grant of the patent: **08.08.2018**
(21) Application number: 13801188.7
(22) Date of filing: 07.06.2013
(51) Int. Cl.: A61N 1/39, B66B 1/46, B66B 1/14

(54) **AUTOMATED EXTERNAL DEFIBRILLATOR FOR ELEVATOR AND METHOD FOR CONTROLLING SAME**
AUTOMATISIERTER EXTERNER DEFIBRILLATOR FÜR AUFZUG UND STEUERUNGSVERFAHREN DAFÜR
DÉFIBRILLATEUR EXTERNE AUTOMATISÉ POUR UN ASCENSEUR ET PROCÉDÉ PERMETTANT DE COMMANDER CE DERNIER

(30) Priority: 07.06.2012 KR 20120060743
(43) Date of publication of application: 15.04.2015
(73) Proprietor: Mediana Co., Ltd., Wonju-si, Gangwon-do 220-801 (KR); Kim, Eong Sok, Gangwon-do 220-885 (KR); Lee, Sung Ho, Gangwon-do 220-884 (KR); Kang, Dong Won, Gangwon-do 220-042 (KR)
(72) Inventor: KIM, Eong Sok, Gangwon-do 220-885 (KR); LEE, Sung Ho, Gangwon-do 220-884 (KR); KANG, Dong Won, Gangwon-do 220-042 (KR)
(74) Representative: Gulde & Partner
(86) International application number: PCT/KR2013/005029
(87) International publication number: WO 2013/183959

(56) References cited:
- JP-A- 2008 265 898
- KR-A- 20110 129 544
- KR-B1- 101 025 295
- US-A- 5 593 426
- US-A1- 2004 055 828
- US-A1- 2004 055 828
- US-A1- 2009 240 297

## Description

### [Technical field]

The present invention relates to an automated external defibrillator (AED) system of elevator and a control method thereof. In more details, AED system of elevator is provided with an AED on the elevator ceiling, and AED call switch beside the entrance of the elevator of each floor, and has an elevator made to move to the floor at which the AED call switch is selected (i.e., pushed), after stopping the current operation when the AED call switch of a certain floor is chosen (i.e., pushed).

### [Background Art]

While deaths due to heart attack (cardiac standstill) are on increase every year, the survival rates of the heart attack patients remain currently at a mere few percent in Korea. Currently, the only equipment available for survival of heart attack patients is Automated External Defibrillator (AED).

AED is an apparatus capable of performing cardiopulmonary resuscitation under guidance by voice, and is configured to be automatically applied electric shocks by checking patient's heart conditions.

Since blood circulation to entire body is stopped when a heart attack occurs, death or serious brain damages can occur unless immediate actions are not taken. Particularly, the brain can be permanently damaged by interruption of blood supply for only 4∼5 minutes.

Cardiopulmonary resuscitation (CPR) is an emergency treatment method to artificially circulate blood and help respiration when heart attacks (cardiac standstill) occurred, and CPR should be performed as the emergency treatment when cardiac arrests occur with abrupt stopping of the patient's heart. Whereas the general CPR recovers about 20% of a normal blood flow rate, use of AED allows 100% recovery, and hence AED can increase the patients' survival rates up to 70% as a maximum by recovering heart beats to normality.

Since the survival rates are dropped by about 10% per minute after occurrence of cardiac standstill, CPR and AED must be used without 5 minutes being exceeded. Therefore, accessibility of an AED is very important for increase in survival rates.

The first place of deaths is a family house, with about 60% of heart attacks occurring at a house. However, equipping every house with the AED which is still high-priced is difficult, and thus plans for most efficiently installing the AED in public housings with densely populated households are desired.

Proposed in the present invention is an AED system of elevators and a control method thereof.

However, when the AED is installed on an elevator, collision or catching can occur upon use of the elevator by people in a narrow space, and much investment is required particularly when the AED is installed on a wall, as disclosed in US 2004/0055828 A1, since the elevator affects the overall line structure in motion.

Consequently, the AED is installed on the elevator ceiling in the present invention so that there will be no collision or catching upon use of the elevator by people in a narrow space with proposition of an AED system of elevators and a control method having a relatively low cost.

Particularly, in the present invention, access by children can be prevented by positioning the handle at an upper part, and the storage box structure has a dustproof effect for long-term storage.

### [Disclosure]

### [Technical problem]

Accordingly, the present invention is aimed to provide an AED system of elevators and control method of it that it is equipped with an AED on the elevator ceiling, and also equipped with AED call switch beside the entrance of the elevator of each floor, and has an elevator made to move to the floor at which the AED call switch is chosen (ON), after stopping the current operation when the AED call switch of a certain floor is chosen.

The present invention is also aimed to provide an AED system of elevators and control method of it that more than one terminal among a terminal of the guard room, a terminal of administration office, an emergency rescue (911 in US or 119 in Korea) terminal, mobile phones of related personnel are automatically informed of the situation when the AED call switch is chosen (ON) and the AED is taken out, in the elevator system equipped with an AED.

The present invention is also aimed to provide an AED system of elevators and control method of it that the AED storage box is equipped with AED inspection unit, and the AED inspection unit regularly check up for normal operation status of the AED.

### [Technical Solution]

To achieve the above aim, the AED system and the control method according to the appended claims are provided. In particular, the AED system of an elevator in the present invention comprises: an AED call switch provided on the side of the entrance door of an elevator or in a key input unit of home automation; an elevator wherein pushing of the AED call switch causes an AED call signal to be transmitted to the elevator; wherein the elevator is adapted to move to the relevant floor at which the AED call switch is pushed when the AED call signal (i.e., a key pushing signal of AED) is received from the AED call switch; the system further comprising an AED storage box which is equipped on the elevator and stores the AED in it, and which turns on the LED on one side of it when the AED call signal is received by the elevator.

The AED call switch transmits the AED call signal to the elevator operating server and the elevator operation server transmits the AED call signal to a terminal of administration office, while the elevator operating server transmits the AED call signal to the relevant elevator.

The terminal of the administration office transmits the AED call signal to a terminal of the guard room or pre-designated mobile phones.

The elevator makes a speaker output a voice informing the emergency situation, when the elevator receives the AED call signal from the AED call switch, and the AED storage box is stored on the elevator ceiling.

AED call switch is provided with a transparent plastic cover at the top, and the plastic cover is to be broken for use when the AED call switch is pushed.

An LED display unit is provided on the bottom face of the AED storage box, and the LED display unit is configured to be visible from within the elevator when the AED storage box is stored on the elevator ceiling.

AED storage box is installed to the upper part of the side of the elevator door on the elevator ceiling.

The AED system of the present invention further comprising:
a support bar which is installed on the top of the AED storage box, and further comprising a spring connecting with the support bar and which is mounted on the main path positioned within the elevator ceiling; and
a handle of the AED storage box,
installed on the bottom of AED storage box, and positioned on an elevator sidewall.

A lock release button is installed on the lower part of handle of AED storage box, and releases the lock that makes the AED storage box be positioned in the ceiling.

The AED storage box is provided with a cover of AED storage box having a transparent window, and provided with an opening/closing handle with a groove underneath the cover of the AED storage box.

AED storage box has a built-in AED inspection unit, and the AED inspection unit regularly checks up for normal operation status of the AED. The AED system may further comprise an LED display unit, wherein the LED display unit includes 4 LED display parts showing whether or not there is an AED, whether normal operation is possible as an automated diagnosis result of AED, and inspection results of the AED inspection unit are transmitted to a terminal of administration office or a terminal of AED administrator. The present disclosure also includes a control method of the AED system of an elevator, wherein the AED storage box having AED is equipped on an elevator, and the elevator moves to the floor that is generated the AED call signal, when the elevator receives the AED call signal from the AED call switch of the floor, has comprising: the elevator moves to the floor generating the AED call signal according to receiving the AED call signal when the elevator is in stopped condition; and the elevator stops current operation, and moves to the floor generating the AED call signal, according to receiving the AED call signal, when the elevator is in operating condition. The storage box has a LED on one side of it and turns on the LED when the AED call signal is received from the elevator.

The elevator door is opened for 2 minutes when the elevator moved to the floor generating the AED call signal (i.e., the floor with the AED call switch pushed), and the elevator door is opened for another 2 minutes when the opening button is pushed additionally.

When the AED call switch is opened and when the AED is taken out, more than one terminal among a terminal of guard room, a terminal of administration office, an emergency rescue terminal, and mobile phones of related personnel receives automatically the situation information.

The elevator is further provided with an LCD display unit which outputs a guide message informing of the emergency situation or pictures describing how to take out AED upon receiving AED call signal from the AED call switch, and the LCD display unit is mounted to the handle for the AED storage box positioned beneath the bottom face of the AED storage box.

When the elevator is moving to the floor generating the AED call signal, the elevator outputs a guide message that informs of moving to the floor generating the AED call signal by using the speaker or the LCD display unit, and also, the elevator outputs pictures describing how to take out AED by using the LCD display unit.

The handle for the AED storage box is folded on the bottom face of the AED storage box by the handle hinge part, and the handle fixing support part (i.e., the fixing support part of the handle) is released when AED call switch is pushed, and the handle of AED storage box is unfolded to come down.

The handle for the AED storage box is able to move up and down in a through hole within the handle fixture installed the bottom face of the AED storage box, and the handle fixing support part is released when AED call switch is chosen, and the handle of AED storage box is came down.

The hinge part of support bar is rotated by pulling the handle of AED storage box that came down, and AED storage box is came down by rotating the hinge part of support bar, and the hinge part of support bar is formed with one side of support bar of AED storage box and the hinge bar crossing the hole of ceiling.

AED call switch is provided with a transparent cover at the top, the transparent cover is configured to be opened by a slide method.

The AED storage box can be mounted to one side of the inner wall of the elevator, or to a bottom part of the elevator, or to a corner part inside the elevator.

### [Advantageous effects]

According to the AED system of elevator of the present invention since AED is provided on the elevator ceiling and AED call switches are provided within each household, the elevator stops current operation and moves to the floor with the AED call switch pushed for when the AED call switch within a household is pushed. Thus AED can be used efficiently without requiring the high-priced AED to be equipped by every household.

Also, the AED is installed on the elevator ceiling in the present invention, collision or catching is prevented when people use the elevator in a narrow space.

In addition, the present invention for elevators provided with AED's has a configuration where more than one of a terminal of guard room, a terminal of administration office, a emergency rescue terminal, and mobile phones of related personnel are automatically informed of the situation when the AED call switch is pushed and when the AED is taken out so that more prompt responses to emergency situations are made possible.

Furthermore, the present invention for an elevator system provided with AED is designed to use normal AED whenever emergency situations occur since the AED storage box has a built-in AED inspection unit, and the AED inspection unit is configured to regularly check up for normal operation status of AED.

In particular, the present invention placed a position of the handle in the upper part so that access by children can be prevented while there is a dust proofing effect for long-term storage due to a structure of the storage box.

### [Brief Description of Drawings]

Figure 1 is an explanation drawing to describe the AED call switch provided on the side of an elevator door in the AED system of an elevator of the present invention.
Figure 2 is an explanation drawing to describe the AED call switch provided in a key input unit for in-house home automation in each household in the AED system of an elevator of the present invention.
Figure 3 is an explanation drawing to describe the AED storage box provided within an elevator in the AED system of an elevator of the present invention.
Figure 4 is an explanation drawing to describe how to drive the AED storage box.
Figure 5 is an explanation drawing to describe the LED display unit on the bottom face of the AED storage box in the AED system of an elevator of the present invention.
Figure 6 is a block diagram to describe how to control the AED system of an elevator of the present invention.
Figure 7 is a flow chart to describe how to control the AED system of an elevator of the present invention
Figure 8a shows a case where the LCD display unit is added to the AED system of an elevator in Figure 3.
Figure 8b is an explanation drawing to describe that the handle of the AED storage box is used in Figure 8a.
Figure 9 is an explanation diagram to describe the structure that lay the AED storage box (150) in the ceiling according to an embodiment example of the present invention.
Figure 10 describes how to take out the AED storage box from the ceiling by using an opening/closing method by a hinge as another embodiment example of the present invention.
Figure 11 is an explanation drawing to describe the AED call switch (AED call button) provided in an elevator according to an embodiment example of the present invention.
Figure 12 is an example for the case where the AED storage box is mounted to a wall inside an elevator as another embodiment example of the present invention.
Figure 13 shows an example for the case where the AED storage box is mounted to the bottom part of an elevator as another embodiment example of the present invention.
Figure 14 shows an example for the case where the AED storage box is mounted to a corner part inside an elevator as another embodiment example of the present invention.

### [Best Mode]

The present invention relates to an AED system of elevators and control method of it that it is equipped with an AED on the elevator ceiling, and also equipped with AED call switch beside the entrance of the elevator of each floor, and has an elevator made to move to the floor at which the AED call switch is chosen (ON), after stopping the current operation when the AED call switch of a certain floor is chosen (pushed).

In the AED system of an elevator according to an embodiment of the present invention, the AED call switch (130) is equipped on the upper part of the elevator switch unit (120) on the side of the elevator door (110).

When the AED call switch (130) is chosen (pushed), the elevator stops current operation and moves first to the floor where the AED call switch (130) chosen (pushed) is positioned. Also, simultaneously with pushing of the AED call switch(130), information on pushing (choosing) of the AED call switch (130) is transmitted to a terminal of administration office, a terminal of guard room, designated mobile phones, emergency rescue phone (911 in US or 119 in Korea).

When the patient with occurrence of cardiac fibrillation occurred in an apartment house, the elevator stops current operation and moves first to the floor with the AED call switch (130) pushed when a person (family of the patient) exit the front door and push the AED call switch (130) installed at the elevator entrance. The AED call switch is provided with a thin and transparent plastic cover on the top. The thin and transparent cover is broken, and then, the AED call switch (130) is pushed. The administration office replaces the broken cover with a new one later.

When the AED call switch (130) is pushed, the passengers on the elevator are informed of the emergency situation by blinking of LED or sound (voice), and the current condition is automatically transmitted to a terminal of administration office, a terminal of guard room, and more than two designated mobile phones.

### [Mode for Invention]

In the following, the AED system of an elevator and a control method thereof of the present invention will be described in detail with reference to the attached drawings.

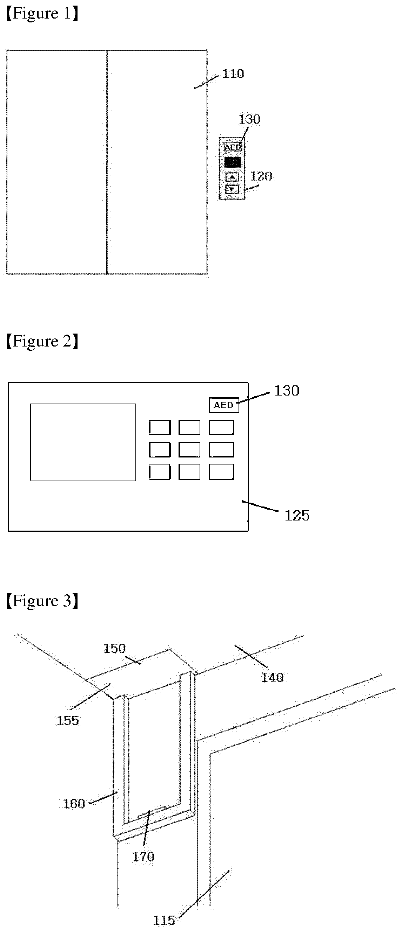

Figure 1 is an explanation drawing to describe the AED call switch provided on the side of an elevator door in the AED system of an elevator of the present invention.

The AED call switch (130) is installed on (the upper part of) the elevator switch unit (110) on the side of the elevator door (110) of an elevator.

When the AED call switch (130) is pushed, the elevator stops current operation and moves first to the floor where the pushed AED call switch (130) is positioned. Also, simultaneously with pushing of the AED call switch (130), informing information for pushing of the AED call switch (130) is transmitted to a terminal of administration office, a terminal of guard room, designated mobile phones, and emergency rescue phone (911 in US or 119 in Korea).

In the present invention, the AED storage box (150) is configured as a structure capable of having a dust proof effect for long-term storage, provided with communication functions, and can prevent access by children by placing position of the handle of the AED storage in the upper part. By installing the AFD on the elevator ceiling (140), there is no collision or catching upon use of the elevator by people in a narrow space. In the present invention, the AED storage box (150) is sealed by packing with rubber material, etc. on the rim of opening part of the AED storage box so as to be provided with a dust proof effect for long-term storage.

Cover for the AED call switch (130) can be discarded after being used once for replacement with a new one.

Namely, when a patient with occurrence of cardiac fibrillation occurred in an apartment house, the elevator stops current operation and moves first to the floor with the AED call switch (130) pushed when a person (family of the patient) exit the front door and push the AED call switch installed at the elevator entrance. The AED call switch is configured so that the AED call switch (130) is pushed as the thin and transparent plastic cover is broken. The administration office replaces the broken cover with a new one later.

When the AED call switch (130) is pushed, passengers on the elevator are informed of the emergency situation as the LED on the bottom face (155) of AED storage box (i.e., bottom of the AED storage box) blinks. Also, when the AED call switch (130) is pushed, the current condition is automatically transmitted to a terminal of administration office, a terminal of guard room, more than two designated mobile phones.

Figure 2 is an explanation drawing to describe the AED call switch provided in the key input unit for in-house home automation of each household in the AED system of an elevator of the present invention.

There is the AED call switch (130) in the key input unit (125) for Home Automation installed inside a house of each household, and the elevator stops current operation, moves first to the floor with the AED call switch (130) pushed when a user pushes the AED call switch (130). Accordingly, the user goes outside by opening the front door, pushes the AED call switch, and waits for the elevator.

When the AED call switch (130) is pushed, passengers on the elevator are informed of the emergency situation as the LED on the bottom face (155) of the AED storage box blinks. Also, the current condition is transmitted automatically to a terminal of administration office, a terminal of guard room, and more than two designated mobile phones.

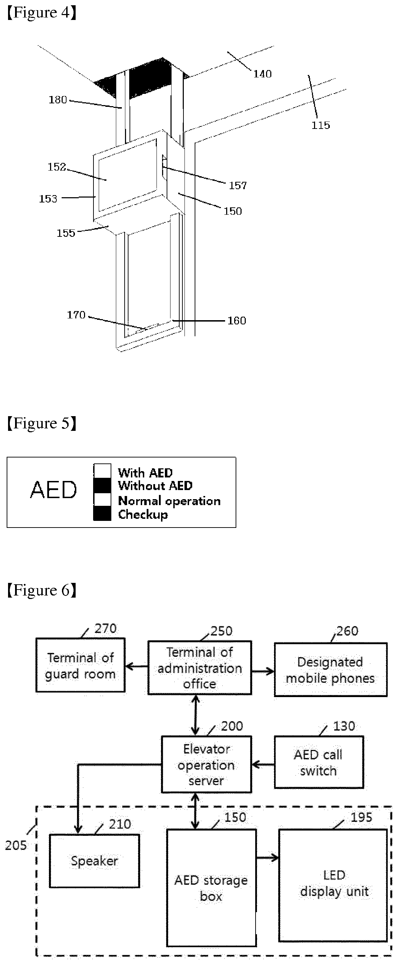

Figure 3 is an explanation drawing to describe the AED storage box provided inside an elevator in the AED system of an elevator of the present invention, while Figure 4 is an explanation drawing to describe how to drive the AED storage box in Figure 3.

The AED storage box (150) is a box to put and keep the AED in it, and is installed in the upper part on the elevator ceiling (140), on the side of the elevator door (115). LED is installed on the bottom face (155) of the AED storage box (150), and is automatically lighted when the AED call switch (130) is pushed.

The AED storage box cover (153) is provided with the transparent window (152), and with the opening/closing handle (157) on one side.

The opening/closing handle (157) is formed together with a groove on the side face of the AED storage box (150).

The handle (160) of AED storage box is installed on one side (a part adjoining the elevator wall) of the bottom face of the AED storage box (150), and is provided with the lock release button (170) on the lower face of the handle (160) of AED storage box. The user moves down the AED storage box (150) by pulling down the handle (160) of AED storage box while pushing the lock release button (170) to release the lock.

The lock release button (170) is positioned in the lower part of the handle (160) of AED storage box, and releases the lock for positioning of the AED storage box (150) within the elevator ceiling (140). Accordingly, the AED storage box (150) comes down outside the elevator ceiling (140) from within the elevator ceiling (140).

The support bar (180) of AED storage box is installed in a rail (not shown) on the main path within the elevator ceiling (140). Namely, one end of the support bar (180) is connected to the top of AED storage box (150), and the other end of the support bar (180) is connected to one end of a spring (not shown). The other end of the spring is installed in the rail.

Depending on cases, a groove (or threshold) can be provided on one side of the support bar (180) of AED storage box, and fixed to the threshold (or groove) on a rail or to (border of) the through-hole part of the ceiling, and the support bar (180) is fixed when the AED storage box (150) comes down outside the elevator ceiling (140). Also, depending on cases, the support bar (180) of AED storage box can be configured in a folding type.

Namely, in the present invention, the AED storage box hiding in the ceiling (140) comes down softly along the rail by spring tension when the handle (160) is pulled down while the lock release button (170) on the handle is being pushed in a situation such as Figure 3. Then, the AED can be taken out by opening the AED storage box (150). The AED storage box (150) can safely protect the AED from dust, etc., and the present invention prevents fast dropping of the AED storage box with a spring and rail structure.

Although Figure 3 and Figure 4 describe coming down of the AED storage box (150) by a spring and a rail, it does not limit the present invention, but can be provided with various methods such as folding type, etc.

As the AED storage box (150) of the present invention is connected by wired/wireless communication, the AED storage box (150) senses the situation where the AED is taken out, and the current condition is automatically transmitted to a terminal of administration office, a terminal of guard room, and more than two designated mobile phones.

When the handle (160) of AED storage box is pushed up after the AED is taken out, the AED storage box (150) goes into the ceiling (140), and is locked to prevent falling down due to shocks and vibrations.

Figure 5 is an explanation drawing to describe the LED display unit on the bottom face of AED storage box in the AED system of an elevator of the present invention.

When the AED call switch (130) is pushed, current elevator passengers are informed through guide broadcasting, and also 'AED' letters are blinking. Through 4 LED lamps, it is displayed whether or not there is AED, whether normal operation is possible as a result of periodic automated diagnosis of AED, and whether checkups are required.

Within the AED storage box (150), AED automatically checks up normal operation status of the system on daily, weekly, monthly bases. The AED transmits these results wirelessly (IRDA, Bluetooth, Wi-Fi , etc.) to the storage box, shows the situation by lighting of LED in the LED display unit on the bottom face (155) of the AED storage box, and also transmits to a terminal of administration office or a server operated by the administration entity (terminal of AED administrator).

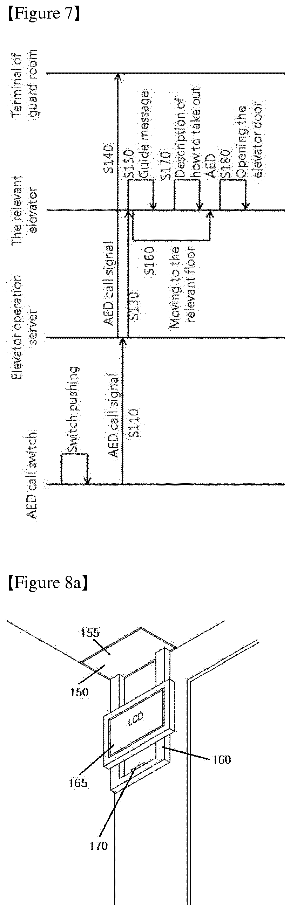

Figure 6 shows a configuration of the AED system of an elevator of the present invention, while Figure 7 is a flow chart to describe how to control the AED system of an elevator of the present invention.

In the present invention, an additional LCD display unit (165) can be provided.

As the step S110, when the AED call switch (130) is pushed at the elevator door (110)(i.e., elevator entrance) or in the key input unit (125) for home automation, AED call signal (i.e., key pushing signals) of the AED call switch (130) is transmitted to the elevator operation server (200), and as the step S130, the elevator operation server (200) transmits the AED call signals to the relevant elevator (205).

Also, as the step S140, the elevator operation server (200) transmits AED call signals (key pushing signals) of the AED call switch (130) to the terminal (250) of administration office, and the terminal (250) of administration office transmits the AED call signals to the terminal (270) of guard room and more than two designated mobile phones (260).

As the step S150, upon receiving of the AED call signals (i.e., key pushing signals) of the AED call switch (130), the relevant elevator (205) informs this fact through the speaker (210), simultaneously turns on the LED display unit (195) and outputs a guide message through the LCD display unit (195), while making the relevant elevator (205) to move to the floor with occurrence of key pushing signals of the AED call switch (130) in step 160.

Namely, passengers aboard will move while recognizing the emergency situation by the speaker (210), the LED display unit (195) and the LCD display unit (195). Also, as the step S170, during moving, a method of taking AED out, etc. are outputted through the LCD display unit (195) and the speaker. Through the same, a passenger aboard can take out the AED first so as to give the AED for more prompt emergency treatment when the door is opened.

As the step S180, after the relevant elevator (205) has moved to the floor generating the AED call signal, namely the floor with the AED call switch (130) pushed, the elevator door will remain opened for 2 minutes, and is opened for additional 2 minutes when the door opening button is pushed additionally. The floor generating the AED call signal is the relevant floor with occurrence of the emergency situation.

When the relevant elevator (205) is in a stopped condition, it will move to the floor generating the AED call signal at a maximum speed, the elevator door will be opened for 2 minutes, and repeat opening of the door for additional 2 minutes whenever the opening button is pushed.

If the current elevator door (115) is open when another floor button is pushed while the relevant elevator (205) is in a stopped condition, the elevator door will be closed, followed by moving to the floor generating the AED call signal.

When the relevant elevator (205) is in operation, implementation of the existing operation will be stopped, and the elevator will move to the floor generating the AED call signal as a normal speed, followed by opening of the door for 2minutes, and repeat opening of the door for additional 2 minutes whenever the opening button is pushed. Although time interval for door opening is recorded as 2 minutes in herewith, it was performed for convenience of explanation rather than to limit the present invention, and it does not matter whatever the time interval may be.

When the situation is concluded and the closing button pushed, the relevant elevator (205) calculates the floor which has to go by using the current position and the stopped situation, and then moves to the floor.

Currently, too high costs are incurred socially for the AED to be installed in every family. Therefore, the AED is being disseminated to allow joint use.

When an elevator is provided with the AED system of the present invention, survival rates of a patient can be increased since accessibility is generally much faster than when the AED is furnished on one first floor of an apartment house, outside an apartment house, in guard room, in administration office.

As a separate button of the key input unit (125) for home automation and the another separate button positioned on the side of the elevator door (110) (i.e., elevator entrance), the AED call switch (130) is provided, and the elevator is made to operate to the floor generating the AED call signal (i.e., the floor with the AED call switch pushed) after the existing operation is cancelled, when the AED call switch (130) is pushed. Therefore, accessibility of the AED call switch (130) is further enhanced.

In the AED system of an elevator of the present invention, inconvenience upon usual operation has been eliminated even inside a narrow elevator by installing the AED above the ceiling.

Also, in the present invention, the handle (160) of AED storage box is arranged at a position beyond the reach of children's hands, and it employed a structure prevented that the AED storage box falls down quickly when the handle of the AED storage box is pull down.

Also, in the present invention, installation of the storage box with a dust-proof structure allows protection of the equipment from dust.

The present invention has a system that is transmitted automatically the situation information to a terminal of guard room, a terminal of administration office, an emergency rescue terminal, and mobile phones of related personnel, when the AED call switch is chosen(ON) and when AED is taken out. Therefore, the present invention allows effective responses to emergency situations.

In the present invention, the AED storage box (150) receives the self test results from the AED, and transmits the self test results to the terminal (250) of administration office through the elevator operation server (200). Thereby, it can be maintained remotely to the breakdown of the system and to the replacement periods of pads and batteries.

In the present invention, it is equipped with LED display unit on the bottom face (155) of the AED storage box. The LEDs of LED display unit display whether AED is or not in the AED storage box, and whether AED is out of order or not.

Depending on cases, the LED display unit (195) on the bottom face (155) of the AED storage box is always lighted, and LEDs of the LED display unit (195) repeatedly turn on and turn off in emergency situations that the AED call switch (130) is pushed, so as to inform the emergency situation.

In the present invention, passengers will be informed through the LED display unit (195) on the bottom face of storage box and voice guide of the speaker (210) when the elevator cancels the existing operation, and performs emergency operation (having more higher priority).

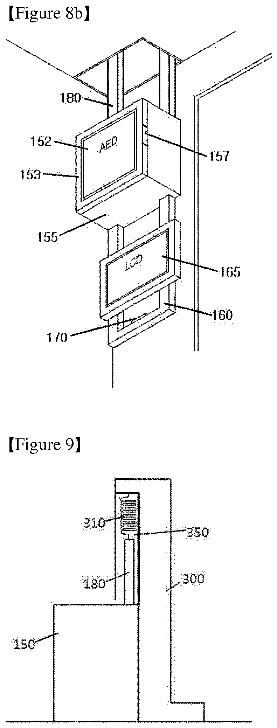

Figure 8a shows the case where an LCD display unit is added to the AED system of an elevator in Figure 3, while Figure 8b is an explanation drawing to describe that the handle of the AED storage box is used in Figure 8a.

The LCD display unit (165) can be installed on the handle (160) of the AED storage box positioned below the bottom face of the AED storage box (150). The LCD display unit (165) outputs a guide message together with voice at inside of the elevator, and the guide message is informed that the elevator currently moves to the floor generating an AED call signal according to an emergency situation. Otherwise, the LCD display unit (165) displays the explanation about the method that take out the AED from the AED storage box (150).

Namely, when the AED call switch (130) outside an elevator is pushed, an announcement of voice is in the elevator, and the announcement is informed that the elevator is currently moving to the floor with the AED call switch (130) pushed according to emergency situation.

If an LCD screen is installed together, it guides to be emergency situations by using image as well as voice. Then, the passenger of the elevator takes out the AED keeping in the elevator, and the passenger gives the AED when the elevator door is opened. Thereby, it can be prepared for emergency situations competing for time.

Figure 9 is an explanation drawing to describe the structure that lay the AED storage box (150) in the ceiling according to an embodiment example of the present invention.

A main path that the support bar (180) of the AED storage box is moved in, is formed on one side of the support (300) in the ceiling, and the main path be provided with the rail (350).

One end of the spring (310) is connected to the top of the support (300) in the ceiling and the other end of the spring (310) is connected to the top of the support bar (180) of the AED storage box, and the bottom of the support bar (180) of the AED storage box is connected to the top of the AED storage box (150).

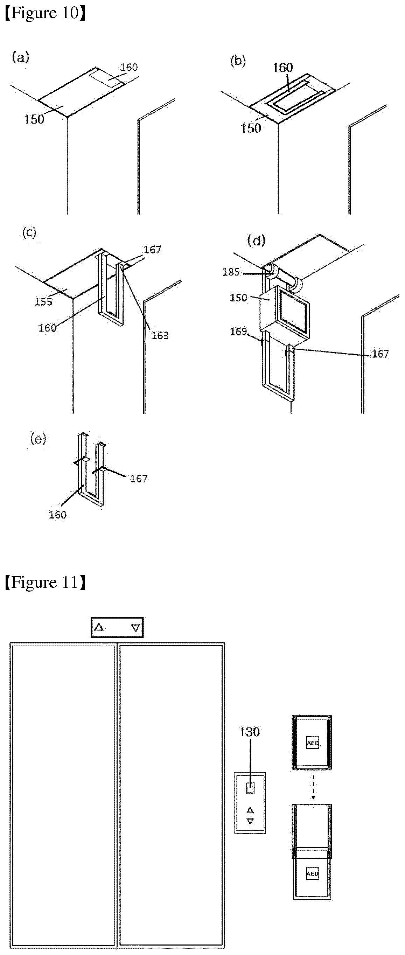

Figure 10 describes how to take out the AED storage box from the ceiling by using an opening/closing method by a hinge, as another embodiment example of the present invention.

The AED storage box (150) is installed in the ceiling as in Figure 10 (a) or Figure 10 (b). The handle (160) of the AED storage box (150) automatically unfolded as in Figure 10 (c) when the AED call switch (130) is pushed. On the other hand, the handle (160) of the AED storage box (150) unfolded automatically when the AED call switch (130) is pushed, and the handle (160) of the AED storage box is pulled down, and the hinge part (185) of support bar are rotated to be opened, and the AED storage box (150) comes down as a result as in Figure 10 (d). The hinge part of support bar is formed with one side of support bar of AED storage box and the hinge bar crossing the hole of ceiling.

Figure 10 (a) shows a condition where the AED storage box (150) is mounted to the ceiling, and the handle (160) is rotatable connected by the handle hinge part (163) while being connected with the handle fixture (167) installed on the bottom of the AED storage box (150). The handle of AED storage box is able to move up and down within the through hole of the fixture (167) as in Figure 10(e).

As the handle (160) of AED storage box is pulled or the AED call switch (130) is pushed, the handle-fixing support part (i.e., the fixing support part of the handle) (not shown) is released, and then, the handle (160) of AED storage box comes down. The handle hinge part (163) is a hinge part formed between one end of the handle (160) and the handle fixture (167).

Figure 10 (b) shows a condition where the handle (160) of AED storage box is folded by the handle hinge part (163) on the bottom face of the AED storage box (150). The handle-fixing support part (not shown) is released when the handle (160) is pulled down and when the AED call switch (13) is pushed, and then, the handle (160) is unfolded and comes down. The handle-fixing support part fixed and supported the handle (160).

When the AED storage box (150) comes down by pulling the handle (160), the handle (160) is fixed to the handle fixture (167) by operating the handle stopper (169).

Figure 11 is an explanation drawing to describe the AED call switch (AED call button) provided for an elevator according to an embodiment example of the present invention.

The AED call switch (130) is positioned on one side of the entrance part of an elevator, and a transparent cover is positioned on the AED call switch (130), and the transparent cover can be configured to be opened by a slide method.

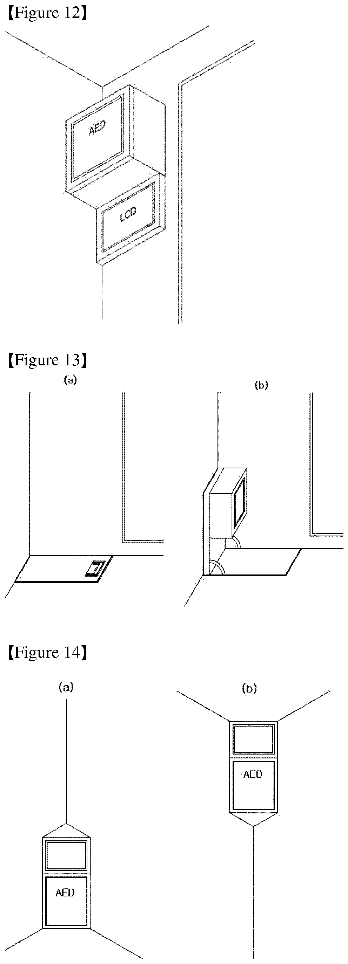

Figure 12 is an example for the case where the AED storage box is mounted to a wall inside an elevator as another embodiment example of the present invention.

With reference to Figure 12, the AED storage box (150) is installed in one side of inside walls of an elevator, and the LCD display unit (165) can be installed below it.

Figure 13 is an example for the case where the AED storage box is installed in the bottom of an elevator as another embodiment example of the present invention.

While the AED storage box (150) is installed in the bottom of an elevator as in Figure 13, the AED storage box (150) is taken out through opening/closing by the hinge part.

Figure 14 shows an example for the case where the AED storage box is mounted to a corner part inside an elevator as another embodiment example of the present invention.

The AED storage box is mounted to a corner part inside an elevator as in Figure 14, and the LCD display unit can be installed on the top of it.

### [Industrial Applicability]

Since the present invention can be applied to automated external defibrillator (AED) systems applicable to elevators and elevators provided with an AED system, every house is not required to be provided with high-priced AED's, and AED's can be used more efficiently by regularly checking up for normal operation status of AED in the elevator systems provided with the AED.

## Claims

1. An automated external defibrillator (AED) system of an elevator comprising:
an AED call switch (130), provided on the side of an elevator door (110) or provided in a key
input unit (125) of a home automation;
an elevator;
wherein pushing of the AED call switch causes an AED call signal to be transmitted to the elevator; wherein the elevator is adapted to move to the floor at which the AED call switch (130) has been activated when an AED call signal is received from the AED call switch (130); the system further comprising
an AED storage box (150), which is equipped in the elevator and stores the AED in it, and turns on a LED on one side of it when the AED call signal is received by the elevator.

2. The AED system of the elevator as claimed in claim 1, wherein the AED call switch (130) transmits the AED call signal to an elevator operation server (200), and the elevator operation server (200) transmits the AED call signal to a terminal (250) of an administration office.

3. The AED system of the elevator as claimed in claim 2, wherein the elevator operation server (200) transmits the AED call signal to the elevator.

4. The AED system of the elevator as claimed in claim 2, wherein the terminal (250) of the administration office transmits the AED call signal to a terminal (270) of a guard room or a predesignated mobile phone (260).

5. The AED system of the elevator as claimed in claim 1, wherein the elevator makes a speaker (210) output a voice informing the emergency situation when the elevator receives the AED call signal from the AED call switch (130).

6. The AED system of the elevator as claimed in claim 1, wherein the AED storage box (150) is stored in the elevator ceiling (140).

7. The AED system of the elevator as claimed in claim 1, wherein AED call switch (130) is provided with a transparent plastic cover on the top, and, the plastic cover is configured to be broken for use when the AED call switch (130) is pushed.

8. The AED system of the elevator as claimed in claim 6, wherein an LED display unit
(195) is provided on the bottom face (155) of AED storage box (150), and the LED display unit (195) is configured to be visible from inside the elevator when the AED storage box (150) is stored in the elevator ceiling (140).

9. The AED system of the elevator as claimed in claim 8, wherein the AED storage box (150) is installed to the upper part of the side of an elevator door (115) on the elevator ceiling (140).

10. The AED system of the elevator as claimed in claim 9, comprising:
a support bar (180), which is installed on the top of the AED storage box (150), and further comprising a spring (310) connecting with the support bar and which is mounted on a main path positioned within the elevator ceiling (140); and
a handle of the AED storage box (150), installed on the bottom of the AED storage box (150), and positioned on an elevator sidewall.

11. The AED system of the elevator as claimed in claim 10, wherein a lock release button (170) is installed on the lower part of the handle (160) of the AED storage box (150), and releases a lock that makes the AED storage box (150) be positioned within the elevator ceiling (140).

12. The AED system of the elevator as claimed in claim 1, wherein the AED storage box (150) is provided with a cover (153) of AED storage box having a transparent window (152), and being provided with an opening/closing handle (157) with a groove underneath the cover (153) of the AED storage box (150).

13. The AED system of the elevator according to any of claims 1 to 12, wherein the AED storage box (150) has an inspection unit, which regularly checks up for normal operation status of the AED.

14. The AED system of the elevator as claimed in claim 13, further comprising an LED display unit and wherein the LED display unit
(195) includes 4 LED display parts indicating a status that the AED is in the AED storage box (150), a status that the AED is not in the AED storage box (150), a status that it is decided for the AED to do the normal operation as a result of automated diagnosis for AED, and a status that it is decided that the AED does not the normal operation as a result of automated diagnosis for the AED, and a check of the AED is needed.

15. The AED system of the elevator as claimed in claim 13, wherein the inspection results of the AED inspection unit is transmitted to the terminal (250) of the administration office or a terminal of an AED administrator.

16. The AED system of the elevator according to any of Claim 1 or Claim 5, wherein the elevator is further provided with a LCD display unit (165) which outputs a guide message (S150) informing the emergency situation or outputs pictures describing how to take out the AED upon receiving the AED call signal from the AED call switch.

17. The AED system of the elevator as claimed in claim 16, wherein the LCD display unit (165) is mounted to a handle (160) of the AED storage box (150) positioned underneath the bottom face (155) of AED storage box (150).

18. The AED system of the elevator as claimed in claim 8, wherein a handle (160) of the AED storage box (150) is folded on the bottom face (155) of AED storage box (150) by a handle hinge part (163), and a handle fixing support part is released when the AED call switch (130) is chosen, and the handle (160) of the AED storage box (150) is unfolded to come down.

19. The AED system of the elevator as claimed in claim 8, wherein a handle (160) of the AED storage box (150) is able to move up and down inside through a hole within a handle fixture (167) installed on the bottom face (155) of the AED storage box (150), and wherein, when the AED call switch is pushed, a handle fixing support part is released
and the handle (160) of the AED storage box (150) comes down.

20. The AED system of the elevator as claimed in claim 1, wherein the AED call switch (130) is provided with a transparent cover, and the transparent cover is configured to be opened by a slide method.

21. The AED system of the elevator as claimed in claim 1, wherein the AED storage box (150) is installed in one side of the inside wall of the elevator.

22. The AED system of the elevator as claimed in claim 1, wherein the AED storage box (150) is installed in the bottom of the elevator.

23. The AED system of the elevator as claimed in claim 1, wherein the AED storage box (150) is mounted to the corner part inside the elevator.

24. A control method of an automated external defibrillator (AED) system of an elevator, wherein an AED storage box having an AED is equipped in an elevator, wherein, in the method, the elevator moves to the floor on which is generated an AED call signal, when the elevator receives the AED call signal from an AED call switch (120) of the floor, further wherein, in the method: the elevator moves to the floor generating the AED call signal according to receiving the AED call signal when the elevator is in stopped condition; and
the elevator stops current operation, and moves to the floor generating the AED call signal, according to receiving the AED call signal, when the elevator is in operating condition,
**characterized in that**
the storage box (150) has a LED on one side of it and turns on the LED when the AED call signal is received by the elevator.

25. The method as claimed in claim 24, wherein the elevator door (110) is opened for 2 minutes (S180), at the time that the elevator moves to the floor generating the AED call signal, and the elevator door is opened for additional 2 minutes when the door opening button is pushed additionally (S180).

26. The method according to claim 24 or 25, wherein more than one terminal among a terminal (270) of a guard room, a terminal (250) of an administration office, an emergency rescue terminal, and mobile phones (260) of related personnel receives the situation information (S140), when the AED call switch (130) is chosen(ON) and when the AED is taken out.

27. The method as claimed in claim 24, wherein the elevator outputs a guide message (S150) that informs of moving to the floor generating the AED call signal, by using a speaker or an LCD display unit (165), when the elevator is moving to the floor generating the AED call signal.

28. The method according to any of claim 27, wherein the elevator outputs pictures (S150) describing how to take out the AED, by using the LCD display unit (165), when the elevator moves to the floor generating the AED call signal.

## Patentansprüche

1. Ein automatisiertes externes Defibrillatorsystem (AED-System) eines Aufzugs, umfassend:
einen AED-Rufschalter (130), der an der Seite einer Aufzugtür (110) vorgesehen ist oder in einer Tasteneingabeeinheit (125) einer Hausautomation vorgesehen ist;
einen Aufzug;
wobei durch Drücken auf den AED-Rufschalter bewirkt wird, dass ein AED-Rufsignal an den Aufzug übertragen wird;
wobei der Aufzug so ausgelegt ist, dass er zu der Etage fährt, in der der AED-Rufschalter (130) betätigt wurde, wenn ein von dem AED-Rufschalter (130) kommendes AED-Rufsignal empfangen wird; wobei das System ferner aufweist:
einen AED-Aufbewahrungskasten (150), der in dem Aufzug vorgesehen ist und den AED darin aufnimmt und eine LED auf einer Seite davon einschaltet, wenn das AED-Rufsignal vom Aufzug empfangen wird.

2. Das AED-System des Aufzugs wie in Anspruch 1 beansprucht, wobei der AED-Rufschalter (130) das AED-Rufsignal an einen Aufzugbetriebsserver (200) überträgt, und der Aufzugbetriebsserver (200) das AED-Rufsignal an ein Endgerät (250) eines Verwaltungsbüros überträgt.

3. Das AED-System des Aufzugs wie in Anspruch 2 beansprucht, wobei der Aufzugbetriebsserver (200) das AED-Rufsignal an den Aufzug überträgt.

4. Das AED-System des Aufzugs wie in Anspruch 2 beansprucht, wobei das Endgerät (250) des Verwaltungsbüros das AED-Rufsignal an ein Endgerät (270) eines Wärterraums oder ein vorbestimmtes Mobiltelefon (260) überträgt.

5. Das AED-System des Aufzugs wie in Anspruch 1 beansprucht, wobei der Aufzug einen Lautsprecher (210) eine Sprachinformation ausgeben lässt, die über die Notfallsituation informiert, wenn der Aufzug das AED-Rufsignal von dem AED-Rufschalter (130) empfängt.

6. Das AED-System des Aufzugs wie in Anspruch 1 beansprucht, wobei der AED-Aufbewahrungskasten (150) in der Aufzugdecke (140) untergebracht ist.

7. Das AED-System des Aufzugs wie in Anspruch 1 beansprucht, wobei der AED-Rufschalter (130) oben mit einer durchsichtigen Kunststoffabdeckung versehen ist und die Kunststoffabdeckung so ausgelegt ist, dass sie für den Gebrauch zerstört wird, wenn auf den AED-Rufschalter (130) gedrückt wird.

8. Das AED-System des Aufzugs wie in Anspruch 6 beansprucht, wobei eine LED-Anzeigeeinheit (195) auf der Unterseite (155) des AED-Aufbewahrungskastens (150) angeordnet ist, und die LED-Anzeigeeinheit (195) so ausgelegt ist, dass sie vom Inneren des Aufzugs aus sichtbar ist, wenn der AED-Aufbewahrungskasten (150) in der Aufzugdecke (140) aufgenommen ist.

9. Das AED-System des Aufzugs wie in Anspruch 8 beansprucht, wobei der AED-Aufbewahrungskasten (150) am oberen Teil der Seite einer Aufzugtür (115) an der Aufzugdecke (140) montiert ist.

10. Das AED-System des Aufzugs wie in Anspruch 9 beansprucht, umfassend:
eine Haltestange (180), die oben auf dem AED-Aufbewahrungskasten (150) montiert ist, und ferner umfassend eine Feder (310), die mit der Haltestange verbunden ist und die an einer Hauptstrecke befestigt ist, die innerhalb der Aufzugdecke (140) platziert ist;
sowie
einen Griff des AED-Aufbewahrungskastens (150), der am Boden des AED-Aufbewahrungskastens (150) montiert und an einer Aufzugseitenwand platziert ist.

11. Das AED-System des Aufzugs wie in Anspruch 10 beansprucht, wobei ein Entriegelungsknopf (170) am unteren Teil des Griffs (160) des AED-Aufbewahrungskastens (150) montiert ist und eine Verriegelung entriegelt, die dafür sorgt, dass der AED-Aufbewahrungskasten (150) innerhalb der Aufzugdecke (140) platziert ist.

12. Das AED-System des Aufzugs wie in Anspruch 1 beansprucht, wobei der AED-Aufbewahrungskasten (150) mit einer Abdeckung (153) des AED-Aufbewahrungskastens versehen ist, die ein durchsichtiges Fenster (152) aufweist und mit einem Griff zum Öffnen/Schließen (157) mit einer Rille unterhalb der Abdeckung (153) des AED-Aufbewahrungskastens (150) versehen ist.

13. Das AED-System des Aufzugs wie in einem der Ansprüche 1 bis 12 beansprucht, wobei der AED-Aufbewahrungskasten (150) eine Prüfeinheit aufweist, die in regelmäßigen Abständen den Normalbetriebszustand des AED überprüft.

14. Das AED-System des Aufzugs wie in Anspruch 13 beansprucht, ferner umfassend eine LED-Anzeigeeinheit und wobei die LED-Anzeigeeinheit (195) vier LED-Anzeigenteile beinhaltet, die einen Zustand angeben, dass sich der AED in dem AED-Aufbewahrungskasten (150) befindet, einen Zustand, dass sich der AED nicht in dem AED-Aufbewahrungskasten (150) befindet, einen Zustand, dass infolge einer automatisierten Diagnose für den AED veranlasst wird, dass der AED normal arbeitet, und einen Zustand, dass infolge einer automatisierten Diagnose für den AED veranlasst wird, dass der AED nicht normal arbeitet und eine Überprüfung des AED erforderlich ist.

15. Das AED-System des Aufzugs wie in Anspruch 13 beansprucht, wobei die Prüfergebnisse der AED-Prüfeinheit an das Endgerät (250) des Überwachungsbüros oder ein Endgerät einer AED-Verwaltungsperson übertragen werden.

16. Das AED-System des Aufzugs nach Anspruch 1 oder Anspruch 5, wobei der Aufzug ferner mit einer LCD-Anzeigeeinheit (165) versehen ist, die nach Empfang des von dem AED-Rufschalters kommenden AED-Rufsignals eine Anleitungsmeldung ausgibt (S150), mit der über den Notfallsituation informiert wird, oder Bilder ausgibt, die beschreiben, wie der AED herauszunehmen ist.

17. Das AED-System des Aufzugs wie in Anspruch 16 beansprucht, wobei die LCD-Anzeigeeinheit (165) an einem Griff (160) des AED-Aufbewahrungskastens (150) befestigt ist, der unter der Unterseite (155) des AED-Aufbewahrungskastens (150) platziert ist.

18. Das AED-System des Aufzugs wie in Anspruch 8 beansprucht, wobei ein Griff (160) des AED-Aufbewahrungskastens (150) mit einem Griffscharnierteil (163) an die Unterseite (155) des AED-Aufbewahrungskastens (150) geklappt ist und ein Grifffixierhalteteil gelöst wird, wenn der AED-Rufschalter (130) gewählt wird und der Griff (160) des AED-Aufbewahrungskastens (150) nach unten abgeklappt wird.

19. Das AED-System des Aufzugs wie in Anspruch 8 beansprucht, wobei ein Griff (160) des AED-Aufbewahrungskastens (150) in der Lage ist, sich durch ein Loch innerhalb eines Griffhalters (167) hindurch, der an der Unterseite (155) des AED-Aufbewahrungskastens (150) montiert ist, nach oben und unten zu bewegen, und wobei beim Drücken auf den AED-Rufschalter ein Grifffixierhalteteil gelöst wird und der Griff (160) des AED-Aufbewahrungskastens (150) herunterkommt.

20. Das AED-System des Aufzugs wie in Anspruch 1 beansprucht, wobei der AED-Rufschalter (130) mit einer durchsichtigen Abdeckung versehen ist und die durchsichtige Abdeckung so ausgelegt ist, dass sie mit einem Schiebeverfahren geöffnet wird.

21. Das AED-System des Aufzugs wie in Anspruch 1 beansprucht, wobei der AED-Aufbewahrungskasten (150) in einer Seite der Innenwand des Aufzugs montiert ist.

22. Das AED-System des Aufzugs wie in Anspruch 1 beansprucht, wobei der AED-Aufbewahrungskasten (150) im Boden des Aufzugs montiert ist.

23. Das AED-System des Aufzugs wie in Anspruch 1 beansprucht, wobei der AED-Aufbewahrungskasten (150) an dem Eckteil innerhalb des Aufzugs befestigt ist.

24. Ein Steuerungsverfahren für ein automatisiertes externes Defibrillatorsystem (AED-System) eines Aufzugs, wobei ein AED-Aufbewahrungskasten mit einem AED in einem Aufzug vorgesehen ist, wobei in dem Verfahren der Aufzug zu der Etage fährt, in der ein AED-Rufsignal erzeugt wird, wenn der Aufzug das von einem AED-Rufschalter (120) der Etage kommende AED-Rufsignal empfängt, wobei ferner in dem Verfahren:
der Aufzug entsprechend dem Empfang des AED-Rufsignals zu der Etage fährt, die das AED-Rufsignal erzeugt, wenn sich der Aufzug im angehaltenen Zustand befindet; und
der Aufzug entsprechend dem Empfang des AED-Rufsignals den gegenwärtigen Betrieb unterbricht und zu der Etage fährt, die das AED-Rufsignal erzeugt, wenn sich der Aufzug im Betriebszustand befindet,
**dadurch gekennzeichnet, dass**
der Aufbewahrungskasten (150) eine LED auf einer Seite davon aufweist und die LED einschaltet, wenn das AED-Rufsignal vom Aufzug empfangen wird.

25. Das Verfahren wie in Anspruch 24 beansprucht, wobei die Aufzugtür (110) zu dem Zeitpunkt, wenn der Aufzug zu der Etage fährt, die das AED-Rufsignal erzeugt, zwei Minuten lang geöffnet wird (S180), und die Aufzugtür weitere zwei Minuten lang geöffnet wird, wenn zusätzlich auf den Türöffnungstaster gedrückt wird (S180).

26. Das Verfahren nach Anspruch 24 oder 25, wobei mehr als ein Endgerät von einem Endgerät (270) eines Wärterraums, einem Endgerät (250) eines Verwaltungsbüros, einem Notfallrettungsendgerät sowie Mobiltelefonen (260) von zugehörigem Personal die Situationsinformationen empfangen (S140), wenn der AED-Rufschalter (130) ausgewählt ist (ON) und wenn der AED herausgenommen ist.

27. Das Verfahren wie in Anspruch 24 beansprucht, wobei der Aufzug unter Verwendung eines Lautsprechers oder einer LCD-Anzeigeeinheit (165) eine Anleitungsmeldung ausgibt (S150), die darüber informiert, dass er zu der Etage fährt, die das AED-Rufsignal erzeugt, wenn der Aufzug zu der Etage fährt, die das AED-Rufsignal erzeugt.

28. Das Verfahren nach Anspruch 27, wobei der Aufzug unter Verwendung der LCD-Anzeigeeinheit (165) Bilder ausgibt (S150), die beschreiben, wie der AED herauszunehmen ist, wenn der Aufzug zu der Etage fährt, die das AED-Rufsignal erzeugt.

## Revendications

1. Système de défibrillateur externe automatisé (DEA) pour un ascenseur, comprenant :
un commutateur d'appel de DEA (130) prévu à côté d'une porte d'ascenseur (110) ou prévu dans une unité de saisie à touches (125) d'une installation domotique ;
un ascenseur ;
où une pression sur le commutateur d'appel de DEA déclenche la transmission d'un signal d'appel de DEA à l'ascenseur ;
où l'ascenseur est prévu pour se déplacer vers l'étage où le commutateur d'appel de DEA (130) a été appuyé quand un signal d'appel de DEA est reçu du commutateur d'appel de DEA (130) ; ledit système comprenant en outre
un boîtier de stockage de DEA (150) monté dans l'ascenseur et recevant le DEA, et allumant une LED sur un de ses côtés quand le signal d'appel de DEA est reçu par l'ascenseur.

2. Système de DEA pour un ascenseur selon la revendication 1, où le commutateur d'appel de DEA (130) transmet le signal d'appel de DEA à un serveur d'exploitation d'ascenseur (200), et où le serveur d'exploitation d'ascenseur (200) transmet le signal d'appel de DEA à un terminal (250) d'un bureau administratif.

3. Système de DEA pour un ascenseur selon la revendication 2, où le serveur d'exploitation d'ascenseur (200) transmet le signal d'appel de DEA à l'ascenseur.

4. Système de DEA pour un ascenseur selon la revendication 2, où le terminal (250) du bureau administratif transmet le signal d'appel de DEA à un terminal (270) d'une salle de gardiennage ou à un téléphone mobile prédéfini (260).

5. Système de DEA pour un ascenseur selon la revendication 1, où l'ascenseur déclenche une émission vocale par un haut-parleur (210), informant de la situation d'urgence quand l'ascenseur reçoit le signal d'appel de DEA du commutateur d'appel de DEA (130).

6. Système de DEA pour un ascenseur selon la revendication 1, où le boîtier de stockage de DEA (150) est monté dans le plafond (140) de l'ascenseur.

7. Système de DEA pour un ascenseur selon la revendication 1, où le commutateur d'appel de DEA (130) est pourvu d'un couvercle en plastique transparent sur le dessus, et où le couvercle en plastique est prévu pour être brisé pour utilisation quand le commutateur d'appel de DEA (130) est appuyé.

8. Système de DEA pour un ascenseur selon la revendication 6, où une unité d'affichage à LED (195) est prévue sur la face inférieure (155) du boîtier de stockage de DEA (150), et où l'unité d'affichage à LED (195) est prévue pour être visible de l'intérieur de l'ascenseur quand le boîtier de stockage de DEA (150) est monté dans le plafond (140) de l'ascenseur.

9. Système de DEA pour un ascenseur selon la revendication 8, où le boîtier de stockage de DEA (150) est monté dans la partie supérieure du côté d'une porte d'ascenseur (115) sur le plafond (140) de l'ascenseur.

10. Système de DEA pour un ascenseur selon la revendication 9, comprenant :
une barre support (180) montée au sommet du boîtier de stockage de DEA (150), et comprenant en outre un ressort (310) raccordé à la barre support et monté sur un trajet principal présenté à l'intérieur du plafond (140) de l'ascenseur ; et
une poignée du boîtier de stockage de DEA (150), montée sur le dessous du boîtier de stockage de DEA (150), et disposée contre une paroi latérale d'ascenseur.

11. Système de DEA pour un ascenseur selon la revendication 10, où un bouton de déblocage (170) est monté sur la partie inférieure de la poignée (160) du boîtier de stockage de DEA (150), et débloque un verrou positionnant le boîtier de stockage de DEA (150) à l'intérieur du plafond (140) de l'ascenseur.

12. Système de DEA pour un ascenseur selon la revendication 1, où le boîtier de stockage de DEA (150) est pourvu d'un couvercle (153) de boîtier de stockage de DEA avec une fenêtre transparente (152), et est pourvu d'une poignée d'ouverture/de fermeture (157) avec une rainure sous le couvercle (153) du boîtier de stockage de DEA (150).

13. Système de DEA pour un ascenseur selon l'une des revendications 1 à 12, où le boîtier de stockage de DEA (150) comporte une unité d'inspection contrôlant régulièrement l'état de fonctionnement normal du DEA.

14. Système de DEA pour un ascenseur selon la revendication 13, comprenant en outre une unité d'affichage à LED, et où l'unité d'affichage à LED (195) comporte 4 sections d'affichage à LED indiquant un état où le DEA est dans le boîtier de stockage de DEA (150), un état où le DEA n'est pas dans le boîtier de stockage de DEA (150), un état où il est décidé que le DEA effectue un fonctionnement normal en tant que résultat de diagnostic automatisé pour le DEA, et un état où il est décidé que le DEA n'effectue pas un fonctionnement normal en tant que résultat de diagnostic automatisé pour le DEA, un contrôle du DEA étant exigé.

15. Système de DEA pour un ascenseur selon la revendication 13, où les résultats d'inspection de l'unité d'inspection de DEA sont transmis au terminal (250) du bureau administratif ou à un terminal d'un administrateur de DEA.

16. Système de DEA pour un ascenseur selon la revendication 1 ou la revendication 5, où l'ascenseur est pourvu en outre d'une unité d'affichage à LCD (165) émettant un message de guidage (S150) informant de la situation d'urgence ou affichant des images décrivant la prise en mains du DEA à la réception du signal d'appel de DEA du commutateur d'appel de DEA.

17. Système de DEA pour un ascenseur selon la revendication 16, où l'unité d'affichage à LCD (165) est montée sur une poignée (160) du boîtier de stockage de DEA (150) placée en dessous de la face inférieure (155) du boîtier de stockage de DEA (150).

18. Système de DEA pour un ascenseur selon la revendication 8, où une poignée (160) du boîtier de stockage de DEA (150) est rabattue sur la face inférieure (155) du boîtier de stockage de DEA (150) au moyen d'une pièce de charnière (163) de poignée, et où une pièce de support de fixation de poignée est débloquée quand le commutateur d'appel de DEA (130) est sélectionné, et la poignée (160) du boîtier de stockage de DEA (150) est rabattue pour descendre.

19. Système de DEA pour un ascenseur selon la revendication 8, où une poignée (160) du boîtier de stockage de DEA (150) peut monter et descendre à l'intérieur d'une ouverture dans une fixation (167) de poignée montée sur la face inférieure (155) du boîtier de stockage de DEA (150), et où, quand le commutateur d'appel de DEA est appuyé, une pièce de pièce de support de fixation de poignée est débloquée, la poignée (160) du boîtier de stockage de DEA (150) descendant alors.

20. Système de DEA pour un ascenseur selon la revendication 1, où le commutateur d'appel de DEA (130) est pourvu d'un couvercle transparent, et le couvercle transparent est prévu pour être ouvert par technique de coulissement.

21. Système de DEA pour un ascenseur selon la revendication 1, où le boîtier de stockage de DEA (150) est monté sur un côté de la paroi intérieure de l'ascenseur.

22. Système de DEA pour un ascenseur selon la revendication 1, où le boîtier de stockage de DEA (150) est monté dans le bas de l'ascenseur.

23. Système de DEA pour un ascenseur selon la revendication 1, où le boîtier de stockage de DEA (150) est monté dans la partie de coin à l'intérieur de l'ascenseur.

24. Procédé de commande d'un système de défibrillateur externe automatisé (DEA) pour un ascenseur, où un boîtier de stockage de DEA pourvu d'un DEA est monté dans un ascenseur, où, dans ledit procédé, l'ascenseur se déplace vers l'étage où est généré un signal d'appel de DEA quand l'ascenseur reçoit le signal d'appel de DEA d'un commutateur d'appel de DEA (120) de l'étage, et où, dans ledit procédé : l'ascenseur se déplace vers l'étage où est généré le signal d'appel de DEA en fonction de la réception du signal d'appel de DEA quand l'ascenseur est en état d'arrêt ; et l'ascenseur arrête le fonctionnement en cours, et se déplace vers l'étage où est généré le signal d'appel de DEA en fonction de la réception du signal d'appel de DEA quand l'ascenseur est en état de fonctionnement, **caractérisé en ce que** le boîtier de stockage (150) a une LED sur un des ses côtés et allume la LED quand le signal d'appel de DEA est reçu par l'ascenseur.

25. Procédé selon la revendication 24, où la porte d'ascenseur (110) est ouverte pendant 2 minutes (S180), au moment où l'ascenseur se déplace vers l'étage où est généré le signal d'appel de DEA, et la porte d'ascenseur est ouverte pour 2 minutes en plus quand le bouton d'ouverture de pote est appuyé en complément (S180).

26. Procédé selon la revendication 24 ou la revendication 25, où plus d'un terminal parmi un terminal (270) d'une salle de gardiennage, un terminal (250) d'un bureau administratif, un terminal d'appel de secours d'urgence, et des téléphones mobiles (260) du personnel concerné reçoivent l'information de situation (S140) quand le commutateur d'appel de DEA (130) est sélectionné (ON) et que le DEA est pris en mains.

27. Procédé selon la revendication 24, où l'ascenseur émet un message de guidage (S150) informant du déplacement vers l'étage où est généré le signal d'appel de DEA, au moyen d'un haut-parleur ou d'une unité d'affichage à LCD (165), quand l'ascenseur se déplace vers l'étage où est généré le signal d'appel de DEA.

28. Procédé selon la revendication 27, où l'ascenseur affiche des images (S150) décrivant comment prendre en mains le DEA, au moyen de l'unité d'affichage à LCD (165) quand l'ascenseur se déplace vers l'étage où est généré le signal d'appel de DEA.
